# NEW EUROPEAN PATENT SPECIFICATION

(11) **EP 0 975 710 B2**
(45) Date of publication and mention of the opposition decision: **10.06.2009**
(45) Mention of the grant of the patent: 10.09.2003
(21) Application number: 98945462.4
(22) Date of filing: 08.10.1998
(51) Int. Cl.: C09K 11/06, C08G 61/02, C07C 22/04

(54) **POLYMER ELECTROLUMINESCENT DEVICE**
POLYMERISCHE ELEKTROLUMINESZENTE VORRICHTUNG
DISPOSITIF ELECTROLUMINESCENT POLYMERE

(30) Priority: 23.10.1997 EP 97203277
(43) Date of publication of application: 02.02.2000
(60) Divisional application: 03075296.8
(73) Proprietor: Merck Patent GmbH, 64293 Darmstadt (DE)
(72) Inventor: SCHOO, Hermannus, F., M., NL-5656 AA Eindhoven (NL); DEMANDT, Robert, NL-5656 AA Eindhoven (NL); LIEDENBAUM, Coen, T., H., F., NL-5656 AA Eindhoven (NL); WIJNBERG, Hans, NL-5656 AA Eindhoven (NL); TEN HOEVE, Wolter, NL-5656 AA Eindhoven (NL); SPOELSTRA, Karin, J., NL-5656 AA Eindhoven (NL)
(86) International application number: PCT/IB1998/001568
(87) International publication number: WO 1999/021936

(56) References cited:
- EP-A1- 0 637 621
- WO-A-98/25874
- WO-A-98/27136
- WO-A1-95/01871
- WO-A1-96/29376
- US-A- 5 558 904
- ADV. MATER., (WEINHEIM, GER.), Volume 10, No. 16, 1998, HUBERT SPREITZER et al., "Soluble Phenyl-Substituted PPVs. New Materials for Highly Efficient Polymer LEDs", pages 1340-1342.
- ADV. MATER., (WEINHEIM, GER.), Volume 10, No. 9, 1998, CHUNG SUNG-JAE et al., "Improved-Efficiency Light-Emitting Diodes Prepared from Organic-Soluble PPV Derivatives with Phenylanthracene and Branched Alkoxy Pendents", pages 684-688.
- POLYMER. PREPR. (AM. CHEM. SOC., DIV. POLYM. CHEM.), Volume 39, No. 1, 1998, HSIEH BING R. et al., "Poly(P-Phenylene Vinylene)s via Precursor Route and Side Chain Route Mothodologies Involving 1, 6-Polymerization", pages 72-73.

## Description

The invention relates to an electroluminescent device comprising an electroluminescent layer disposed between a hole-injecting and an electron-injecting electrode, said electroluminescent layer comprising a soluble poly(1,4-phenylenevinylene). The invention further relates to poly(1,4-phenylenevinylene)s (PPVs) for use in such a device.
The invention also relates to intermediate compounds suitable for the preparation of such poly(1,4-phenylenevinylene)s.
An electroluminescent (EL) device in which light emission originates from a polymer, referred to as polymer EL device for short, can be suitably used as a (pixelated) dis>play device or a lighting device, in particular a large-area lighting device such as a backlight for a liquid crystal display.

A polymer EL device as mentioned in the opening paragraph is known from, for example, the international patent application WO 96/08047. In the known EL device a layer of a soluble poly(2,5-dialkoxy-1,4-phenylenevinylene) is disposed between an indiumtinoxide (ITO) hole-injecting electrode (anode) and an electron-injecting electrode (cathode) of a metal having a low work function such as calcium. When driven at room temperature, the EL device emits orange light during its entire service life.

However, the inventors have observed that when the known EL device is driven after having been kept at an elevated ambient temperature of, for example, 70 °C, the color of the emitted light gradually shifts from orange to red. The wavelength at which the emission intensity is at a maximum may shift as much as 100 nm. In the context of the invention, this phenomenon is referred to as red shift. In the case of polymer EL devices which emit in the orange to red part of the visible spectrum, such as said known EL device, the red shift will furthermore result in a significant part of their emission spectrum being pushed into the infrared range of the electromagnetic spectrum, thus causing their brightness to be reduced in the visible range.

The occurrence of a red shift renders the EL device less suitable for those applications in which it is more or less prescribed that the performance of a device should be substantially unaltered if said device is stored and/or driven at elevated ambient temperatures. Examples of such application include, in particular, automotive applications. It is not uncommon that the interior of an automobile reaches a temperature of 70 °C or more when exposed to direct sun light.

WO96/29376 discloses an electroluminescent device which emits polarized light using stiff-chain oligo-p-phenylene polymers which are soluble in organic solvents.

EP 637621 discloses polymeric fluorescent substances and electroluminescent devices comprising such substances, where the substance may comprise three different poly-phenylene-vinylene units A, B, C having different optical absorption edge wavelengths and are present in the substance in different relative amounts.

WO 95/01871 discloses blends of luminescent polymer in matrix of hole transport polymer where the polymer may be poly(p-phenylphenylene vinylene) and electroluminescent devices comprising such blends. The blending with hole-transporting polymer causes color shift of the luminescent polymer.

It is an object of the invention, inter alia, to overcome or at least reduce the above mentioned disadvantage. Specifically, it is an object of the invention to provide a polymer EL device whose light emitting surface shows, when driven, a reduced red shift after having been kept or driven at an elevated ambient temperature for an extended period of time. The red shift, expressed in terms of the shift of the wavelength at which the emission intensity is greatest, is preferably less than 15 nm or, more preferably, less than 5 nm.

This object is achieved by an EL device as described in the opening paragraph which, in accordance with the invention, is characterized in that at least one 1,4-phenylene unit of said poly(1,4-phenylenevinylene) is an aryl-1,4-phenylene unit, wherein the aryl group represents a phenyl, naphthyl, or biphenylyl group which may or may not be substituted, with the exception of the copolymer poly(2-(3-methoxyphenyl)-1,4-phenylenevinylene -*co*- 2-methoxy-5-(2-(trimethylammonium iodide)-ethoxy)-1,4-phenylenevinylene) and poly(p-phenylphenylene vinylene).

As a typical example, in the case of poly(2-methoxy-5-(3,7-dimethyloctyloxy)-1,4-phenylenevinylene), which exhibits a red shift of about 100 nm, it is found that the red shift can be reduced to a small or negligible value by replacing one half of the 2-methoxy-5-(3,7-dimethyloctyloxy)-1,4-phenylene units by phenyl-1,4-phenylene units.

As another typical example in accordance with the invention, a small or negligible red shift is also observed if substantially all 1,4-phenylene units are aryl-1,4-phenylene units, in other words if the EL polymer is a homopolymer having an aryl-1,4-phenylenevinylene unit as the single repeating unit.

In the context of the invention, the term polymer includes homopolymer, copolymer, terpolymer etc. as well as oligomer.

The invention is based on the observation that the extent of red shift is related to the nature of the EL polymer in the solid state, which is substantiated by the fact that the photoluminescence (PL) spectrum shows a similar red shift when a layer of the EL polymer is subjected to an elevated ambient temperature.

It is noted that in WO 95/01871, an EL device is disclosed wherein the EL layer comprises the homopolymer poly(phenyl-1.4-phenylenevinylene) in which the phenyl group is unsubstituted. Said EL device is not in accordance with the invention since, in the context of the present invention the EL polymer is insoluble, its solubility being at most 0.06 wt. %. In the context of the present invention, a PPV is soluble if its solubility in common organic solvents such as toluene is significantly larger than 0.06 wt. %, i.e. at least 0.5 wt. %. Clear and non-gelled solutions of at least this concentration are required if neat layers thereof are to be applied by spin-coating.

In the non-prepublished international patent application with application number IB97/00366 (PHN 15.772) filed by Applicants, an EL device is disclosed which has an EL layer comprising the copolymer poly(2-(3-methoxyphenyl)-1,4-phenylenevinylene -*co*- 2-methoxy-5-(2-(trimethylammonium iodide)-ethoxy)-1,4-phenylenevinylene). However, said application is completely silent on the phenomenon of red shift, let alone means to suppress such a red shift.

The use of aryl-substituted 1,4-phenylene units reduces the red shift, while causing other characteristics of the EL polymer of which said units are part to be maintained or even improved. In particular, it is found that the use of aryl-substituted 1,4-phenylene units improves the photoluminescence efficiency of the EL polymer of which it is part, whereas its solubility and film-forming ability are similar to or even better than the solubility and film-forming ability of poly(1,4-phenylenevinylene)s which are substituted with other groups. All other relevant factors being equal, an increase in photoluminescence efficiency of an EL material implies that an EL device employing said EL material will emit more light.

Although aryl groups larger than naphthyl and 2-biphenylyl, such as anthracyl, may be used to reduce the red shift, the use of such large aryl groups is not convenient since the ratio of main chain to side chain becomes unfavorably small then. Because the main chain (backbone) of the PPV is deemed to be involved in light emission and charge carrier transport, this ratio is to be preferably large.

The aryl group may or may not be substituted. With respect to its red shift reducing ability, the number and choice of substituents is not critical. The aryl group may be substituted by heteroatoms, a suitable example being a 3-pyridyl group. Suitable substituents include alkyl and alkoxy groups, a (5-phenyl)-3,4-oxadiazolyl group which may increase the electron mobility, and a N,N-dialkylamino or N,N-diphenylamino group which may increase the hole mobility. If necessary, the solubility of the PPV to be used in the EL device in accordance with the invention may be increased by means of alkoxy and/or alkyl groups which are preferably branched and, if more than one such group is present, of unequal length.

The EL layer of the polymer device preferably has a thickness of 50 nm to 200 nm, in particular 100 nm to 175 nm or preferably 80 nm to 150 nm.

The electron-injecting electrode is suitably made of a metal (alloy) having a low work function, such as Yb, Ca, Mg:Ag Li:Al or In.

The hole-injecting electrode is suitably made of a metal (alloy) having a high work function such as Au, Pt, Ag. Preferably, a more transparent hole-injecting electrode material, such as an indiumtinoxide (ITO), is used. Conductive polymers such as a polyaniline and a poly-3,4-ethylenedioxythiophene are also suitable transparent hole-injecting electrode materials. Preferably, the hole-injecting electrode is made of an ITO layer covered on the side of the EL layer with a thin layer of poly-3,4-ethylenedioxythiophene.

A particular embodiment of the EL device in accordance with the invention is characterized in that the aryl-1,4-phenylene unit is a unit of the formula (C1) or (C2) 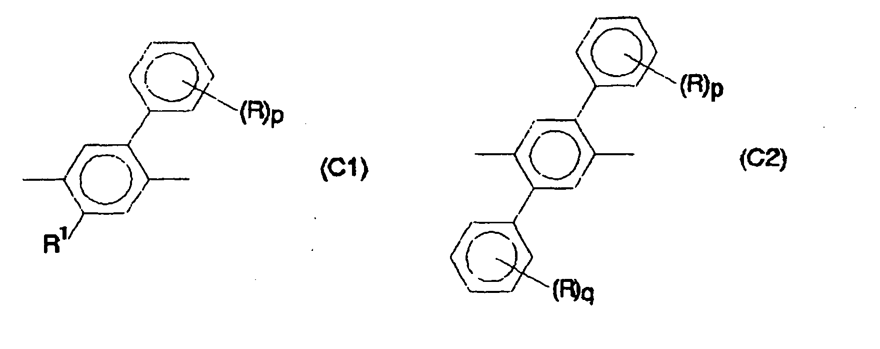 wherein,
R is independently selected each time it occurs and represents C₁-C₂₀ alkyl or C₁-C₂₀ alkoxy, R¹ represents hydrogen or C₁-C₂₀ alkoxy,
p and q are 0, 1, 2, 3, 4 or 5, and
one or more non-neighboring -CH₂- units of a C₁-C₂₀ alkyl or C₁-C₂₀ alkoxy may be replaced by -O-, -S-, -N(R²)-, wherein R² equals phenyl or C₁-C₂₀ alkyl, phenylene, and/or -COO-. As already mentioned above, the homopolymer of units (C1) wherein R¹ equals H and p equals 0 is excluded since it lacks solubility.

An aryl group as small as the phenyl group is already effective in reducing red shift, said phenyl group preferably being provided with substituents in order to, inter alia, enhance the solubility of the poly(1,4-phenylenevinylene) of which it is part. An alkyl and alkoxy group or a heteroatom-substituted analog of said group is particularly effective in this respect. Said groups are preferably branched and, if more than one such group is present, of unequal length.

The solubility does not increase further if alkyl and alkoxy groups larger than C₂₀ are used. In order to increase the ratio of main chain to side chain and thus increase the proportion of atoms directly involved in electroluminescence, a smaller alkyl or alkoxy group, viz. C₁-C₁₂, is preferably used.

Suitable alkyl groups include methyl, ethyl, propyl, dodecyl, 3,7-dimethyloctyl. Suitable alkoxy groups include methoxy, 3,7-dimethyloctyloxy, 2-methylpropoxy.

In order to increase the ratio of main chain to side chain atoms, the number of substituents per phenyl group p or q is to be small, preferably 1 or 2.

A good balance between solubility and the ratio of main chain to side chain is obtained if the total number of non-hydrogen atoms of all substituents R of a phenyl group is about 10.

A particular embodiment is claimed in Claim 3. The poly(2,5-dialkoxy-1,4-phenylenevinylene)s are very suitable EL polymers, yet suffer from a rather large red shift, which moreover causes a significant part of the emission spectrum to be located outside the visible spectrum. If 2,5-dialkoxy-1,4-phenylene units are replaced by aryl-1,4-phenylene units the red shift is effectively reduced while at the same time the properties which render 2,5-dialkoxy PPVs particularly suitable for use in EL devices are at least maintained, if not improved.

The poly(1,4-phenylenevinylene) may comprise one, but preferably more than one, aryl-1,4-phenylene unit, i.e. an aryl-1,4-phenylenevinylene is preferably a repeating unit. The size of the red shift associated with a particular PPV depends on the proportion of aryl-1,4-phenylene units employed, said proportion being defined as the ratio of the number of aryl-1,4-phenylene units to the total number of 1,4-phenylene units of the PPV. It also depends on the nature of the 1,4-phenylene units which do not carry an aryl group. The proportion of aryl-1,4-phenylene units required in order to obtain a PPV having a red shift below a specified value can be simply determined empirically on a case by case basis by varying said proportion of aryl-1,4-phenylene units.

A particular embodiment of the EL device is characterized in that the proportion of aryl-1,4-phenylene units is selected between 0.001 and 0.1. Within this range the emission spectrum is substantially the same as the emission spectrum of the corresponding PPV in which said proportion is 0.0.

Alternatively, if the color of the light emitted by an EL device comprising an aryl-substituted PPV is to be significantly different from that of the light emitted by the corresponding PPV which is not aryl-substituted, the proportion of aryl-1,4-phenylene units is to be selected between 0.1 and 0.95.

A preferred embodiment of the EL device in accordance with the invention is characterized in that the aryl-1,4-phenylene units are present in a proportion of 0.95 to 1.0 inclusive. Polymer EL devices which comprise a PPV in which substantially all 1,4-phenylene units are aryl-1,4-phenylene units emit green light when driven. This is an additional advantage since EL devices which are based on poly(1,4-phenylenevinylene)s and emit green light are few and far between. Moreover, the green light-emitting poly(1,4-phenylenevinylene)s in accordance with the invention are very stable, as is demonstrated by the service life of EL devices made thereof, in particular when the aryl-1,4-phenylene units satisfy the formula (C1) or (C2). For example, a green light emitting EL device in accordance with the invention typically has an efficiency of about 5.0 Cd/A and a service life of more than 1500 h, the service life being determined by the time span during which the brightness drops to half its initial value, while operating the device by continuously adjusting the voltage so as to maintain a constant current.

It is noted that in US 5,247,190 a PPV-based green light-emitting EL device is described. However, said PPV is insoluble and EL layers thereof are made by means of depositing a precursor material. The present invention does not relate to polymer EL devices whose light emission originates from such an insoluble PPV.

In WO 95/32526 an EL device emitting green light and employing a soluble 2,5-dialkyl PPV is described. The present invention does not relate to such PPVs. The same applies to EL devices which employ a PPV which is made to emit green light by deliberately interrupting the conjugation of its main chain by introducing saturated units. The service life of EL devices based on such PPVs is unsatisfactory.

The solubility of the green light-emissive poly(aryl-1,4-phenylenevinylene) may be enhanced by replacing some of the aryl-1,4-phenylene units by solubility enhancing 1,4-phenylene units. A suitable solubility-enhancing unit is a 2,5-dialkoxy-1,4-phenylenevinylene, in particular a 2-methoxy-5-(3,7-dimethyloctyloxy)-1,4-phenylenevinylene or 2,5-bis(3,7-dimethyloctyloxy)-1,4-phenylenevinylene unit. If the fraction of said solubility-enhancing units is less than 0.05 the color of the emitted light is substantially the same as that of the homopolymer.

The invention further relates to poly(1,4-phenylenevinylene)s for use in an EL device and aims to provide novel soluble poly(1,4-phenylenevinylene)s.

In accordance with the invention, this aim is achieved by a soluble poly(1,4-phenylenevinylene) wherein at least one 1,4-phenylene unit is an aryl-1,4-phenylene unit, where the aryl group represents a phenyl, naphthyl, or biphenylyl group which may or may not be substituted, with the exception of the copolymer poly((3-methoxyphenyl)-1,4-phenylenevinylene -*co*- 2-methoxy-5-(2-(trimethylammonium iodide)-ethoxy)-1,4-phenylenevinylene)s and poly (p-phenylphenylene vinylene).

As already mentioned hereinabove, by using a PPV having aryl-1,4-phenylene unit(s) the red shift is reduced in comparison with the corresponding PPV which does not have such units. Particular embodiments of the PPV in accordance with the invention have already been mentioned hereinabove. In general, the PPVs in accordance with the invention can be suitably used as constituents of semiconducting and/or luminescent materials.

Said PPVs can be prepared using synthetic methods similar to methods well known to those skilled in the art and include those disclosed in WO 96/29356. Many methods of preparing a PPV employ a 1,4-bis(halomethyl)benzene as an intermediate compound. In particular, said intermediate compound can be suitably used as a monomer, for example, in a base-catalyzed polymerization of said 1,4-bis(halomethyl)benzenes.

The invention therefore also relates to intermediate compounds suitable for the preparation of poly(1,4-phenylenevinylene)s. If a poly(1,4-phenylenevinylene) in accordance with the invention is to be prepared, said intermediate compound is an aryl-1,4-bis(halomethyl)benzene, wherein the aryl group represents a phenyl, naphthyl, or biphenylyl group which may or may not be substituted, with the exception of 2-phenyl-1,4-bis(bromomethyl)benzene and 2-(3-methoxyphenyl)-1,4-bis(chloromethyl)benzene. An alternative name for 2-phenyl-1,4-bis(bromomethyl)benzene is 2,5- bis(chloromethyl)-1,1'-biphenyl, whereas 2-(3-methoxyphenyl)-1,4-bis(chloromethyl)benzene may also be referred to as 2,5- bis(chloromethyl)-3'-methoxy-1,1'-biphenyl.

These and other aspects of the invention will be apparent from and elucidated with reference to the exemplary embodiments described hereinafter.

In the drawings:
Figure 1 schematically shows a cross-sectional view of a polymer EL device,
Figure 2 shows an emission spectrum of an EL device not in accordance with the invention when driven at room temperature before (curve A) and after (curve B) having been exposed to an elevated ambient temperature,
Figure 3 shows an emission spectrum of an EL device in accordance with the invention when driven at room temperature before (curve A) and after (curve B) having been exposed to an elevated ambient temperature,
Figure 4 shows another emission spectrum of an EL device in accordance with the invention when driven at room temperature before (curve A) and after (curve B) having been exposed to an elevated ambient temperature.

### Example 1

This first example presents a number of embodiments of aryl-1,4-bis(halomethyl)benzene intermediate compounds and methods of preparing such compounds.

### A. Preparation of 2-methyl-2',5'-bis(chloromethyl)-1,1'-biphenyl

### A1. 1,4-bisbromomethyl-2-bromobenzene

A quantity of 2-bromo-p-xylene (500 g, 2.70 mol, made by bromination of p-xylene with bromine in the presence of a trace of iodine at 15 - 20 °C) and 100 ml carbon tetrachloride are heated to 90 °C. Some dibenzoylperoxide is added, followed by the dropwise addition of bromine (320 ml, 6.22 mol) at about 90 °C over a period of about 8 h. From time to time some dibenzoylperoxide is added. The mixture is heated at 85 - 95 °C overnight, then cooled. More carbon tetrachloride is added and the solution is washed with 300 ml water, 300 ml dilute sodium hydroxide, 300 ml water, then dried and rotary evaporated. The residue is stirred with 500 ml hexane containing a small amount of toluene. The solid is filtered off and washed with hexane. It weighs 200 g. The filtrate is rotary evaporated, to the residue is added 250 ml carbon tetrachloride and the solution is refluxed. Some dibenzoylperoxide is added, followed by the portion-wise addition of N-bromosuccinimide (100 g, 0.56 mol). The mixture is refluxed for 3 h, then cooled and washed with water, dilute sodium hydroxide solution and water. After drying and rotary evaporation a residue is obtained which is stirred with hexane and some toluene. The solid is collected and dried, it weighs 64 g. The filtrate is rotary evaporated and then purified through Kugelrohr distillation. The forerun is discarded and the fraction containing much product is collected and, subsequently, stirred with hexane containing some toluene to give another 90 g of pure product. Total yield 354 g (1.03 mol, 38%). ¹H NMR (CDCl₃): 4.3 (s, 2H), 4.5 (s, 2H), 7.1-7.6 (m, 3H).

### A2. 1,4-bis(methoxymethyl)-2-bromobenzene

The quantity of 1,4-bisbromomethyl-2-bromobenzene as prepared in step A1 is added in portions to a solution of sodium (110 g, mol) in 1500 ml methanol (slightly exothermal). The mixture is refluxed for 1 h, then stirred at room temperature (rt) overnight. After rotary evaporation water and toluene are added. The layers are separated and the organic layer is washed with water, dried, rotary evaporated and purified through Kugelrohr distillation. There is obtained 235.77 g of a colorless oil (0.962 mol, 93%). ¹H NMR (CDCl₃): 3.3 (s, 3H), 3.4 (s, 3H), 4.3 (s, 2H), 4.45 (s, 2H), 7.0-7.7 (m, 3H).

### A3. 2-methyl-2',5'-bis(methoxymethyl)-1,1'-biphenyl

A Grignard reagent is made from 80.0 g 2-bromotoluene (0.47 mole) and 11.4 g magnesium (0.47 mole) in 150 ml ether in the usual way. A few drops of 1,2-dibromoethane are added to start up the reaction. When most of the magnesium has reacted, the solution is cooled, decanted and added dropwise to a cooled (0-10 °C) solution of 92 g 1,4-bis(methoxymethyl)-2-bromobenzene as prepared in step A2 and 2.0 g Ni(dppp)₂Cl₂ (3.7 mmole) in 100 ml ether. The reaction is exothermic. The reaction mixture is then brought to rt, kept at rt for 15 min and then refluxed. The progress of the reaction is followed by ¹H NMR. Another 250 mg Ni(dppp)₂Cl₂ is added after 24 h in order to complete the reaction. After refluxing for 2 days, the mixture is cooled, poured onto 250 ml 5% HCl and extracted with ether. The water layer is extracted again with ether (150 ml and 100 ml). The ether layers are washed with brine, combined, dried and evaporated to give a reddish brown oil. ¹H NMR showed that the product contained several compounds. The oil is distilled bulb-to-bulb giving several fractions. The fairly pure fractions (about 120 °C/0.01 mbar) are combined giving 16.8 g product (66 mmol, 14%). ¹H NMR (CDCl₃): 2.0 (s, 3H), 3.2-3.35 (d, 6H), 4.1-4.5 (d, 4H), 6.9-7.65 (m, 7H).

### A4. 2-methyl-2',5'-bis(chloromethyl)-1,1'-biphenyl

The quantity of 2-methyl-2',5'-bismethoxymethyl-1,1'-biphenyl as prepared in A3 is stirred vigorously with 245 ml boiling conc. hydrochloric acid for 24 h. It is then cooled to rt and the water layer is extracted twice with hexane (2x100 ml). The hexane layers are dried and evaporated. The residue is heated again with 150 ml conc. hydrochloric acid for 24 h. The mixture is processed as described hereinabove and the product is heated for 72 h with 150 ml conc. hydrochloric acid. Said processing yields a crude product which is purified by Kugelrohr distillation to give 12.3 g of a colorless oil (70%) at 100 °C/0.003 mbar. ¹H NMR (CDCl₃): 2.1 (s, 3H), 4.25-4.55 (d, 4H), 6.9-7.5 (m,7H).

### B. Preparation of 3-phenyl-2',5'-bis(chloromethyl)-1,1'-biphenyl

### B1. 3-bromodiphenyl

Concentrated hydrochloric acid (120 ml) is added dropwise to a mechanically stirred mixture of m-bromoaniline (103 g, 0.6 mole) and 50 ml water, causing the HCl-salt thereof to precipitate. The mixture is heated and stirred at 100 °C, but remains a suspension. It is then cooled to (0-5 °C) and at this temperature a solution of sodium nitrite (43.1 g, 0.62 mole) in 85 ml water is added. The reaction is exothermic, therefore the sodium nitrite has to be added slowly. The cold diazotized solution is poured into a large flask and cold benzene (750 ml) and tetramethylammonium bromide (3 g) are added. A solution of sodium hydroxide (140 ml, 5 M) is added dropwise over a period of 45 min. The temperature during the reaction is kept at about 5 °C; when all the alkali has been added, the reaction mixture is allowed to warm up slowly to rt overnight. The layers are separated and the upper layer is washed twice with water (2 x 300 ml); the combined aqueous layers are extracted once with some benzene and this is added to the original aqueous layer. The layers are shaken and separated. The combined benzene layers are dried (Na₂SO₄) and evaporated to give a dark brown oil which is distilled at 110 °C/ 0.02 mbar. Yield: 34.0 g (24%) as a yellow oil. ¹H-NMR (CDCl₃): 7.0-8.0 (m, 9H).

### B2. 3-tri-n-butylstannyldiphenyl

A quantity of n-butyllithium (68 ml 2.5 M, 0.17 mole) is added dropwise to a cold (-70 °C) solution of 3-bromodiphenyl as prepared in B1 (34.0 g, 0.15 mol) in 100 ml THF. The yellow solution darkened very much upon addition. After stirring at -70 °C for 15 min, tri-n-butyltin chloride (43 ml, 0.16 mol) is added at this temperature. The reaction mixture is allowed to warm up to rt, stirred overnight at rt and then poured onto water (300 ml). The mixture is shaken and the layers are separated. The organic layer is washed again with water (300 ml). The combined aqueous layers are extracted with hexane (100 ml); the combined hexane layers are dried (Na₂SO₄) and evaporated. The residual oil is distilled bulb-to-bulb giving 49.7 g of 3-tri-n-butylstannyldiphenyl. Yield: 49.7 g (77%) as an oil at 180-200 °C/0.01 mbar. ¹H-NMR (CDCl₃): 0.7-1.8 (m, 27H), 7.4-7.8 (m, 8H).

### B3. 1,1'-diphenyl, 3-phenyl-2',5'-dicarboxylic acid, dimethylester

A solution of 3-tri-n-butylstannyldiphenyl (B2) (49.7 g, 0.11 mol) and 1-bromo-2,5-dicarboxylic acid, dimethylester (30.6 g, 0.11 mol) and Pd(PPh₃)₂Cl₂ (0.9 g, 1.28 mmol) in 150 ml dry THF is refluxed for 7 days during which time more catalyst is added (up to a total of 1.4 g, 2.0 mmol) to speed up the progress of the reaction. The solution is cooled, poured onto 200 ml water and the layers are separated. The aqueous layer is extracted twice with hexane (2 x 100 ml). The product layer is diluted with hexane (200 ml) and washed with water (200 ml). Because no separation occurs, the layers are filtered over Celite. The filtrate can then be separated and the hexane layer is washed with water. The combined hexane layers are dried (Na₂SO₄) and evaporated. Tri-n-butyltin bromide is distilled from the residue, the residue is then introduced into some toluene and filtered through an alumina column. The eluate is evaporated and the resultant product is used. Yield: 27.7 g (72%) as a viscous oil. ¹H-NMR (CDCl₃): * 3.6-3.9 (ss, 6H), 7.2-8.2 (m, 12H).

### B4. 1,1'-diphenyl, 3-phenyl-2',5'-dimethanol

A solution of 27.7 g 1,1'-diphenyl, 3-phenyl-2',5'-dicarboxylic acid, dimethylester (B3) (80 mmol) in 100 ml THF is added dropwise to a cold (0 °C) and mechanically stirred suspension of LiAlH₄ (4.6 g, 120 mmol) in 100 ml THF. During the addition care is taken to keep the temperature below 20 °C. The reaction mixture is then heated at 50 °C for 75 min, cooled to rt and poured onto a mixture of water (200 ml) and toluene (100 ml). The aqueous layer is acidified with concentrated hydrochloric acid after which the layers are shaken and separated. The upper layer is washed with water (200 ml). The combined aqueous layers are extracted once with toluene (100 ml). The combined toluene layers are dried (Na₂SO₄) and evaporated to give 1,1'-diphenyl, 3-phenyl-2',5'-dimethanol which is used unaltered.
Yield: 14.5 (63%) as a light yellow solid. ¹H-NMR (CDCl₃): * 4.4-4.8 (m, 4H), 5.0-5.4 (m, 2H), 7.2-7.9 (m, 12H).

### B5. 3-phenyl-2',5'-bis(chloromethyl)-1,1'-biphenyl.

Thionyl chloride (11 ml, 150 mmol) is added dropwise to a cold (0 °C) suspension of 1,1'-diphenyl, 3-phenyl-2',5'-dimethanol (B4) (14.5 g, 50 mmol) and 0.5 ml pyridine in 60 ml acetonitrile. The suspension is heated at 50 °C for 25 min and becomes clear. The solution is evaporated and the residue is dissolved in 100 ml toluene, washed twice with a dilute base (2 x 50 ml 10% NaOH) and once with water. The toluene layer is dried (Na₂SO₄) and filtered through a short alumina column. *The* eluate is evaporated and the residue is dissolved in hexane and filtered again through a short alumina column. Because recrystallization of the evaporated eluate failed, the resultant crude product is used unaltered. Yield: 8.5 g (52%) as a yellow oil. ¹H-NMR (CDCl₃): * 4.5 (d, 4H), 7.2-7.8 (m,12H).

### C. Preparation of 3-(2-methylpropoxy)-2',5'-bis(chloromethyl)-1,1'-biphenyl

### C1. 3-bromo-1-(2-methylpropoxy)benzene

A mixture of m-bromophenol (34.6 g, 0.2 mol), 125 ml DMSO, coarse-powdered KOH (31 g, 0.47 mole) and isobutylchloride (31 g, 0.47 mol) are stirred, together with 20 g NaBr, for 5 days. More KOH (10 g) is added because ¹H-NMR showed 50 % only conversion. The reaction mixture is stirred for another 5 h, water (10 ml) is added and stirring is continued overnight. After processing (extraction with toluene and water) 3-bromo-1-(2-methylpropoxy)benzene is obtained which can be used unaltered.
Yield: 20.9 g (46%). ¹H-NMR (CDCl₃): 0.9-1.1 (d, 6H), 1.8-2.3 (m, 1H), 3.6-3.7 (d, 2H), 6.6-7.5 (AB, 4H).
Preparatory method steps C2 - C5 are then performed which are similar to B2 - B5, yielding 3(2-methylpropoxy)-2',5'-bis(chloromethyl)-1,1'-biphenyl.
Yield: 8 g (50%) as a yellow oil. ¹H-NMR (CDCl₃): * 0.8-1.2 (d, 6H), 1.7-2.4 (m, 1H), 3.65-3.8 (d, 2H), 4.4-4.6 (ss, 4H), 6.7-7.5 (m, 7H).

### D. Preparation of 3,4-bis(2-methylpropoxy)-2',5'-bis(chloromethyl)-1,1'-biphenyl

### D1. 1,2-bis(2-methylpropoxy)benzene

One half of a solution of 74.4 g (1.86 mole) NaOH in 200 ml water is added dropwise to a solution of 100.0 g (0.908 mole) catechol in 400 ml 96% EtOH. The resulting reaction mixture is refluxed. Sodium iodide (10 g) and triethylbenzylammonium chloride (2 g) are added, followed by isobutylbromide (156.2 g, 1.14 mole) over a period of 3 h. The reaction mixture is refluxed for another 30 min, after which the other half of the NaOH solution is added. A mixture of isobutylchloride (78 g, 0.84 mole) and 45 g isobutylbromide (0.33 mole) is added slowly to the refluxing reaction mixture. It is refluxed for another 5 days after which it is evaporated. The residue is treated with hexane (250 ml) and 10% NaOH solution (250 ml). The layers are separated and the water layer is extracted twice with hexane (2x100 ml). The hexane layers are combined and washed with 10% NaOH solution and with water, dried and evaporated to give 92 g of an orange liquid which is distilled bulb-to-bulb giving 83.2 g (41%) of 1,2-bis(2-methylpropoxy)benzene as a colorless liquid at 90 °C/0.003 mbar). ¹H NMR (CDCl₃): 0.8-1.15 (d, 12H), 1.85-2.5 (m, 2H), 3.65-3.8 (d,4H), 6.85 (s, 4H).

### D2. 1-bromo-3,4-bis(2-methylpropoxy)benzene

N-bromosuccinimide (64.9 g, 0.36 mol) is added in portions to a cooled (0 °C) solution of 1,2-bis(2-methylpropoxy)benzene (73.9 g, 0.33 mol) in 350 ml acetonitrile. During the addition of N-bromosuccinimide more acetonitrile (3x100 ml) has to be added in order to maintain a stirrable reaction mixture. The temperature is then allowed to rise to rt during which time the reaction mixture becomes a clear solution. Stirring is continued for 1.5 h after which the solution is evaporated. The residue is treated with toluene (200 ml) and water (200 ml). The toluene layer is washed once with water and the combined water layers are extracted with a little toluene. The combined toluene layers are dried and evaporated to give 104 g of a reddish brown oil. The oil is crystallized from methanol and a few drops of water at 6 °C. It is filtered in a vacuum and washed with 400 ml methanol/water (9:1). After drying 68.9 g (69%) of 1-bromo-3,4-bis(2-methylpropoxy)benzene is obtained as colorless crystals. ¹H NMR (CDCl₃): 0.8-1.2 (d, 12H), 1.8-2.4 (m, 2H), 3.6-3.8 (d,4H), 6.6-7.1 (m, 3H).
Preparatory method steps D3 - D6 are then performed which are similar to the method steps B2 - B5, yielding 3,4-bis(2-methylpropoxy)-2',5'-bis(chloromethyl)-1,1'-biphenyl.
Yield: 22.4 g (47 %) as a viscous oil. ¹H-NMR (CDCl₃): 1.0-1.2 (dd, 12H), 2.05-2.15 (m, 2H), 3.75-3.85 (d, 4H), 4.5-4.65 (d, 4H), 6.8-7.5 (m, 6H).

### E. Preparation of 4-(2-(5-phenyl-1,3,4-oxadiazolyl))-2',5'-bis(chloromethyl)-1,1'-biphenyl

### E1. 1-benzoyl-2-(4-bromobenzoyl)hydrazine

A quantity of 4-bromobenzoylchloride (25 g, 0.11 mol) and 15.3 g benzhydrazide (0.11 mol) are refluxed in 90 ml pyridine for 30 min. It is then poured onto ice, causing the product to solidify and precipitate. After the ice has melted, the suspension is filtered in a vacuum and the solid is washed with water. The wet cake is dried by stripping with toluene and drying in air. Yield: 33 g (94%) of 1-benzoyl-2-(4-bromobenzoyl)hydrazine.

### E2. 2-(4-bromophenyl)-5-phenyl-1,3,4-oxadiazole

A quantity of 33 g of 1-benzoyl-2-(4-bromobenzoyl)hydrazine (El) (103 mmol) is refluxed in 120 ml POCl₃ for 2 h, in which process the white thin paste becomes a clear brown solution. POCl₃ is distilled off, the residue is poured onto 250 g of crushed ice. The precipated product is filtered in a vacuum and washed with water. After drying in air the crude product is recrystallized from a mixture of ethanol and ethylacetate, resulting in 19.8 g of the product. After rotary evaporation of the filtrate the residue is recrystallized from ethanol/ethylacetate to give another 5.5 g of the product, resulting in a total yield of 25.2 g (84 mmol, 84%), mp 164-166 °C.
Preparatory method steps E3 - E6 are then performed which are similar to the method steps B2 - B5, yielding 4-(2-(5-phenyl-1,3,4-oxadiazolyl))-2',5'-bis(chloromethyl)-1,1'-biphenyl. Yield: 4.3 g (52%.) as a yellow solid. ¹H-NMR (CDCl₃): * 4.5-4.7 (ss, 4H), 7.15-8.3 (m, 12H).
Using similar methods, the following intermediate compounds have been prepared and supplied by Syncom B.V., Groningen, the Netherlands:
3-(3,7-dimethyloctyloxy)-2',5'-bis(chloromethyl)-1,1'-biphenyl,
4-(3,7-dimethyloctyloxy)-2',5'-bis(chloromethyl)-1,1'-biphenyl,
4-(2-methylpropoxy)-2',5'-bis(chloromethyl)-1,1'-biphenyl,
2-methoxy-2',5'-bis(chloromethyl)-1,1'-biphenyl,
3-methoxy-2',5'-bis(chloromethyl)-1,1'-biphenyl,
3-methyl-2',5'-bis(chloromethyl)-1,1,-biphenyl,
4-methyl-2',5'-bis(chloromethyl)-1,1'-biphenyl,
4-(N,N-dimethylamino)-2',5'-bis(chloromethyl)-1,1'-biphenyl,
4-(3-dimethylamino-2-methylpropoxy)-2',5'-bis(chloromethyl)-1,1'-biphenyl,
2-(1-naphthyl)-1,4-bischlorobenzene,
4-(N,N-diphenylamino)-2',5'-bis(chloromethyl)-1,1'-biphenyl,
2-(3-pyridyl)-1,4-bis(chloromethyl)benzene,
4-(3,7-dimethyloctyloxy)-3'-methoxy-2',5'-bis(chloromethyl)-1,1'-biphenyl,
4-(3,7-dimethyloctyloxy)-2',5'-bis(chloromethyl)-4"-(3,7-dimethyloctyloxy)-1,1',4', 1"-terphenyl.

### Example 2

This second example provides embodiments of poly(1,4-phenylenevinylene)s in accordance with the invention which are prepared using intermediate compounds of example 1. The exemplary poly-1,4-phenylenevinylenes satisfy the formula (C3) 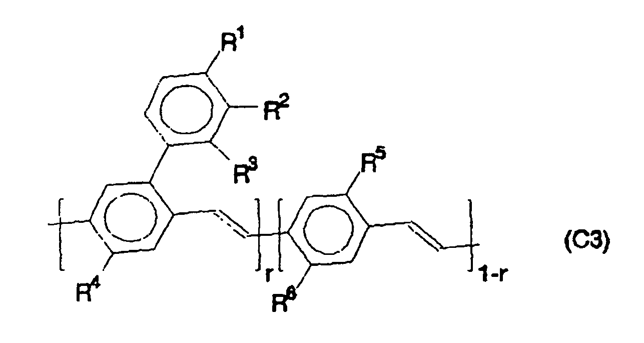 wherein r and 1-r indicate the proportion of 1,4-phenylenevinylene units having the structure indicating in brackets to which, respectively, r and 1-r is suffixed.
By way of example, the polymer which satisfies the formula (C3), wherein r = 1.0, R¹ = R² = 2-methylpropoxy, R³ = R⁴ = H is prepared as follows:

### F. Preparation of poly-(3,4-bis(2-methylpropoxy)phenyl)-1,4-phenylenevinylene (NRS-291)

In a 1000 ml three-neck flask in nitrogen, 3 g of 3-(3,4-isopropyloxy)-1,4-bis(chloromethyl)benzene is dissolved in 750 ml of anhydrous 1,4-dioxane. The solution is heated to 98 °C and 2.2 g of t-BuOK in 100 ml (2.5 eq) of 1,4-dioxane is added slowly in 5 min using a dropping funnel. This solution is allowed to react for 2 min. During addition of the base the color of the reaction mixture turns from colorless to green. Then a solution of 2.5 eq (2.2 g) t-BuOK in 50 ml of 1,4-dioxane is added quickly and allowed to react for 2 h. After 20 min the solution is dark green. After two hours the heating is stopped and the reaction mixture is allowed to cool, or is cooled to 55 °C in one hour. After 3 h the reaction mixture is quenched with 5 ml acetic acid at 55 °C. The acidic solution is stirred for another 0.5 h. The solution becomes bright green. While vigorously stirring, the polymer solution is added slowly to 1000 ml of water and stirred for 30 min. The polymer is filtered, washed with methanol and dried in a vacuum. The polymer is purified by dissolving it in THF (0.75%) and fractionated with 750 ml of methanol (2 times). The obtained polymer is bright green. The yield is 65 mol%. The wavelength at which the photoemission intensity is at a maximum is 550 nm. The polymer is soluble in chloroform, toluene, cyclohexanone, etc. At 30 °C a solution of 0.6 wt. % in toluene remains clear, does not form a gel and can be suitably used to provide neat layers thereof using spin-coating.

### G. Poly-3-(4-(3,7-dimethyloctyloxyphenyl)-1,4-phenylenevinylene(NRS-259)

The polymer NRS-259 is prepared using a method similar to the method provided under F hereinabove. It luminesces in bright green. The wavelength at which the photoemission intensity is at a maximum is 550 nm. The polymer is soluble in chloroform, toluene, cyclohexanone, etc. At 75 °C a solution of 0.6 wt. % in cyclohexanone remains clear, does not form a gel and can be suitably used to provide neat layers thereof using spin-coating.

By using monomer mixtures of different bishalobenzenes in different molar ratios, copolymers, terpolymers etc. can be obtained in any desired ratio.

Using similar methods of preparation, other poly-1,4-phenylenevinylenes in accordance with the invention can be obtained. Further examples satisfying the formula (C3) are listed in Table 1, wherein G₁ = 2-methylpropoxy and G₂ = 3,7-dimethyloctyloxy.

**Table 1**

| polymer | r | R¹ | R² | R³ | R⁴ | R⁵ | R⁶ |
|---|---|---|---|---|---|---|---|
| NRS-249 | 0.80 | H | CH₃ | H | H | OCH₃ | G₂ |
| NRS-258 | 0.87 | H | OCH₃ | H | H | OCH₃ | G₂ |
| NRS-277 | 0.96 | G₂ | H | H | H | OCH₃ | G₂ |
| NRS-251 | 1.00 | H | OCH3 | H | H | - | - |
| RS-270 | 0.00 | - | - | - | - | OCH₃ | G₂ |
| NRS-286 | 0.05 | G₂ | H | H | H | OCH₃ | G₂ |
| NRS-287 | 0.10 | G₂ | H | H | H | OCH₃ | G₂ |
| NRS-288 | 0.25 | G₂ | H | H | H | OCH₃ | G₂ |
| NRS-290 | 0.50 | G₂ | H | H | H | OCH₃ | G₂ |
| NRS-296 | 0.50 | G₁ | G₁ | H | H | OCH₃ | G₂ |
| NRS-259 | 1.00 | G₂ | H | H | H | - | - |
| NRS-250 | 0.93 | H | CH₃ | H | H | OCH₃ | G₂ |
| NRS-280 | 0.97 | H | G₁ | H | H | G₂ | G₂ |
| NRS-285 | 1.00 | H | H | CH₃ | H | - | - |
| NRS-289 | 0.96 | H | H | CH₃ | H | G₂ | G₂ |
| NRS-291 | 1.00 | G₁ | G₁ | H | H | - | - |
| NRS-293 | 1.00 | G₁ | G₁ | H | H | - | - |
| NRS-294 | 0.97 | G₁ | G₁ | H | H | G₂ | G₂ |

The polymers in Table 1 are soluble in toluene, cyclohexanone, chloroform, etc., the solubility being sufficient, that is 0.5 to 1.0 wt. % (corresponding to about 5 to 10 mg/ml), to allow layers thereof to be prepared by means of spin-coating.

### Example 3

### (not in accordance with the invention)

This example presents an embodiment not in accordance with the invention. It serves to demonstrate the problem of red shift associated with the prior art.

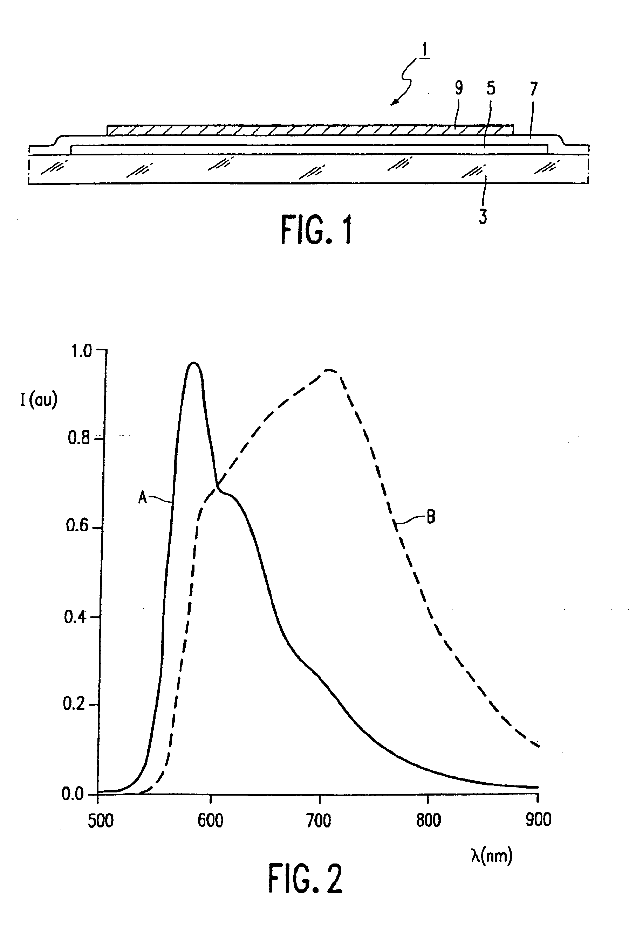

Figure 1 schematically shows a cross-sectional view of a polymer EL device 1 comprising an EL layer 7 disposed between an electron-injecting electrode 9 and a hole-injecting electrode 5 provided on a substrate 3.

In accordance with the international patent application WO 96/08047, a polymer EL device 1 having the following stack of layers is manufactured:
- a glass substrate 3 transparent to the light to be emitted;
- a hole-injecting electrode 5 consisting of, successively, a 150 nm thick ITO layer and a 120 nm thick layer of poly-3,4-ethylenedioxythiophene;
- a 130 nm thick EL layer 7 of poly(2-methoxy-5-(3,7-dimethyloctyloxy)-1,4-phenylenevinylene), in table 1 identified as RS-270;
- a 250 nm thick electron-injecting electrode layer 9 of calcium.

In an inert atmosphere, the EL device 1 is connected to a direct voltage source, the negative pole being connected to the cathode 9. The EL device is then driven at room temperature by applying a voltage of 3 V upon which a light-emitting surface emerges instantly having an orange color and a luminance of 20 Cd/m². The emission spectrum of the light which emerges from the EL device 1 is recorded. This emission spectrum is represented in Figure 2 as curve A. In Figure 2, the emission spectrum is represented as the intensity I (in arbitrary units, au) as a function of wavelength λ (in nm). The EL device 1 is then exposed to an elevated ambient temperature of 125 °C during 25 s. Upon application of a voltage of 3 V a red light-emitting surface emerges. Its emission spectrum is recorded and is represented as curve B in Figure 2.

Referring to Figure 2, it is clearly observed that curve B is red-shifted with respect to curve A. In particular, the wavelength at which the emission intensity is at a maximum has shifted from 595 nm to 720 nm, a red shift of more than 100 nm.

### Example 4

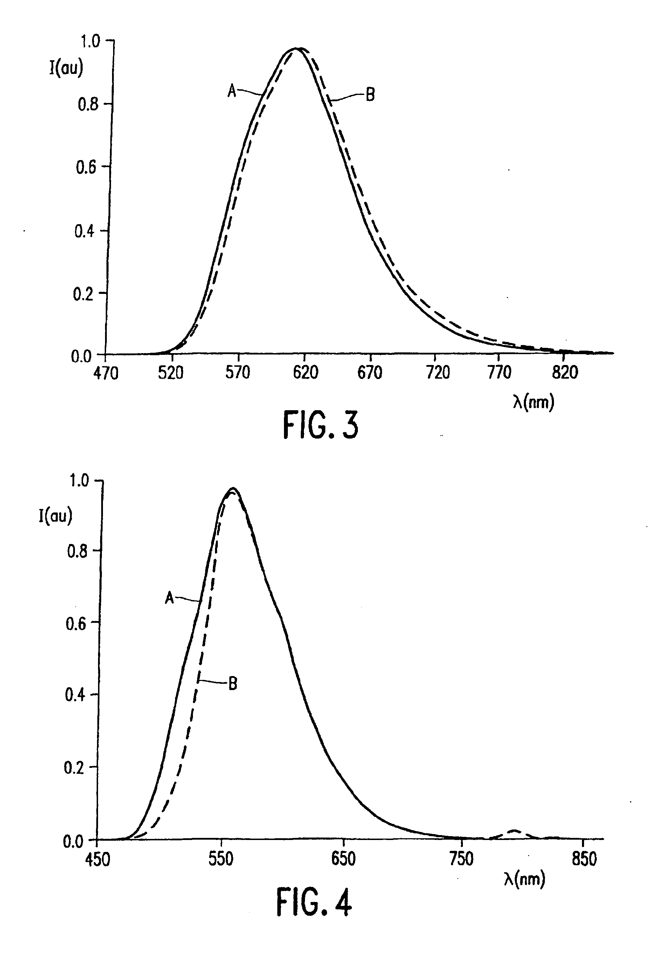

Example 3 is repeated with this difference that the EL layer 7 in the present example consists of a 100 nm thick layer of the polymer NRS-290 (cf. Table 1) which is a polymer in accordance with the invention. The layer is manufactured by means of spin-coating a 0.6 wt. % solution in toluene. Upon application of a voltage of 3 V, an orange light-emitting surface emerges instantly. The resulting emission spectra are given in Figure 3.

Figure 3 shows an emission spectrum of the EL device in accordance with the invention when driven at room temperature before (curve A) and after (curve B) having been exposed to an elevated ambient temperature of 125 °C for 25 s.

The two emission spectra are substantially coincident. A red shift is absent.

Using a fresh specimen of the EL device in accordance with the invention the service life is determined by measuring the time span during which the brightness drops to half its initial value of 20 Cd/m², while operating the device by continuously adjusting the voltage so as to maintain a constant current. The service life thus obtained exceeds 5000 h. Within this time span the emission spectrum remains substantially unaltered.

### Example 5

Example 3 is repeated with this difference that use is made of the homopolymer identified in Table 1 as NRS-291, which is prepared as described in example 2 under F. Said polymer luminesces in bright green. The resulting emission spectra are given in Figure 4.

Figure 4 shows an emission spectrum of the EL device in accordance with the invention when driven at room temperature before (curve A) and after (curve B) having been exposed to an elevated ambient temperature of 125 °C for 25 s.

Referring to Figure 4, it is observed that, in accordance with the invention, an EL device comprising an EL polymer substantially consisting of aryl-1,4-phenylenevinylene units does not exhibit a red shift if it is subjected to an elevated ambient temperature. The small change at the short wavelength side of the emission peak is a negligible change which cannot be discerned by the unaided human eye.

### Example 6

In a further example of an EL device emitting green light when driven, the EL device of example 3 is modified in that in the present example the EL layer 7 consists of a 91 nm thick layer of the polymer identified as NRS-250 in Table 1, said layer being applied by means of spin-coating a solution of said polymer in toluene.

When driven at a voltage of 3 V, the EL device emits green light at a luminance of 20 Cd/m². The service life at room temperature exceeds 1500 h. The color of the light emitted remains unaltered when the EL device is subjected to an elevated ambient temperature of 125 °C for 25 s or 70 "C for several hours.

### Example 7

Of a number of poly-1,4-phenylenevinylenes presented in Table 1 (cf. example 2) a solid film is made by spin-coating. Subsequently, the photoluminescence emission spectrum of each of these solid films is measured before and after the solid film has been subjected to an elevated ambient temperature of 70 °C for 1 h. From the emission spectrum obtained before and after the elevated temperature treatment, the wavelength at which the photoluminescence emission intensity is at a maximum is determined and designated as λ_{max,before} respectively λ_{max,after}, respectively. The difference between these two quantities defines the red shift Δλ.

Subsequently, of each of the solid films the photoluminescence efficiency is measured by means of a calibrated integrating sphere, the measured value being designated as PL-Eff. The results are summarized in Table 2 in which r has the same meaning as in Table 1. For comparison, Table 2 also lists the results obtained for the polymer from exemplary embodiment 2, RS-270, which is a polymer not in accordance with the invention.

**Table 2**

| compound | r | λ_{max,betore} (nm) | λ_{max,after} (nm) | Δλ (nm) | PL-Eff (%) |
|---|---|---|---|---|---|
| NRS-249 | 0.80 | 580 | 580 | 0 | 33 |
| NRS-258 | 0.87 | 565 | 565 | 0 | 38 |
| NRS-277 | 0.96 | 540 | 540 | 0 | 20 |
| NRS-251 | 1.00 | 540 | 540 | 0 | 24 |
| RS-270 | 0.00 | 595 | 650 | 55 | 8 |
| NRS-286 | 0.05 | 595 | 650 | 55 | 9 |
| NRS-287 | 0.10 | 595 | 640 | 45 | 7 |
| NRS-288 | 0.25 | 595 | 630 | 35 | 7 |
| NRS-290 | 0.50 | 600 | 600 | 0 | 14 |
| NRS-296 | 0.50 | 590 | 590 | 0 | 13 |
| NRS-259 | 1.00 | 550 | 550 | 0 | 30 |

The results in Table 2 demonstrate that the red shift is reduced if some of the 1,4-phenylene units are aryl-1,4-phenylene units. Also, if all 1,4-phenylene units are aryl-1,4-phenylene units the red shift is absent or very small.

Furthermore, it is found that PPVs having 2-aryl-1,4-phenylene units show a wide range of emission colors including green (λₘₐₓ roughly between 500 nm and 530 nm) yellow (λₘₐₓ roughly between 530 and 570 nm) and orange to red (λₘₐₓ roughly between 570 and 650 nm).

Table 3 provides further examples of poly(1,4-phenylenevinylene)s in accordance with the invention. It demonstrates that introducing 2-aryl-1,4-phenylene units increases the photoluminescence efficiency. All other factors being equal, this implies that an EL device in which such a polymer is employed will emit more light.

**Table 3**

| compound | λ_{max,before} (nm) | PL-Eff (%) |
|---|---|---|
| NRS-250 | 550 | 41 |
| NRS-280 | 560 | 41 |
| NRS-281 | 540 | 31 |
| NRS-285 | 525 | 24 |
| NRS-289 | 530 | 40 |
| NRS-291 | 560 | 38 |
| NRS-293 | 560 | 52 |
| NRS-294 | 560 | 35 |

## Claims

1. An electroluminescent device comprising an electroluminescent layer disposed between a hole-injecting and an electron-injecting electrode, said electroluminescent layer comprising a soluble poly(1,4-phenylenevinylene), **characterized in that** at least one 1,4-phenylene unit of said poly(1,4-phenylenevinylene) is an aryl-1,4-,phenylene unit, wherein the aryl group represents a phenyl, naphthyl, or biphenylyl group which may or may not be substituted, with the exception of the copolymer poly(2-(3-methoxyphenyl)-1,4-phenylenevinylene-co-2-methoxy-5-(2-(trimethylammonium iodide)-ethoxy)-1,4-phenylenevinylene) and poly(p-phenylphenylene vinylene),
and with the exclusion of
poly(2-methoxy-5-(3,7-dimethyloctyloxy)-p-phenylenevinylene)co-(2-(4'-(3,7-dimethyloctyloxy)phenyl)-p-phenylenevinylene),
poly(2-methoxy-5-(3,7-dimetbyloctyloxy)-p-phenylenevinylene)co-(2-(3'-(3,7-dimethyloctyloxy)phenyl)-p-phenylenevinylene),
poly(2-methoxy-5-(3,7-dimethyloctyloxy)-p-phenylenevinylene)co-(2-(2',5'-dimethyl)phenyl)-p-phenylenevinylene),
poly(2-methoxy-5-(3,7-dimethyloctyloxy)-p-phenylenevinylene)co-(2-(3'-(3,7-dimethyloctyloxy)phenyl)-p-phenylenevinylene)co-(2-(2',5'-dimethyl)phenyl)-p-phenylenevinylene),
poly(2,5-bis(3,7-dimethyloctyloxy)-p-phenylenevinylene)co(2-(3'-(3,7-dimethyloctyloxy)phenyl)-p-phenylenevinylene)phenylenevinylene)co(2-(4'-(3,7-dimethyloetyloxy)phenyl)-p-phenytenevinyJene)phenylenevinylene),
poly(2-(3,7-dimetbyloctyloxy)-5-methoxy-p-phenylenevinylene)co(2-(3'-(3,7-dimethyloctyloxy)pbenyt)-5-methoxy-p,phenytenevinylene),
poly(2-(3'-(3,7-dimethyloctyloxy)phenyl)-p-phenylenevinylene)co(2-(4'-(3,7-dimethyloctyloxy)phenyl)-p-phenylenevinylene),
poly(2-(4'-(3,7-dimethyloctyloxy)phenyl)-p-phenylenevinylene)co(2-(2',5'-dimethyl)phenyl)-p-phenylenevinylene),
poly(2-methoxy-5-(3,7-dimethyloctyloxy)-p-phenylenevinylene)co(2-(3'-(3,7-dimethyloctyloxy)phenyl)-p-phenylenevinylene)phenylenevinylene)co(2-(4'-(3,7-dimethyloctyloxy)phenyl)-p-phenylenevinylene)co(2-(2',5'-dimethyl)phenyl)-p-phenylene,
poly(2-(3'-(3,7-dimethyloctyloxy)phenyl)-p-phenylenevinylene)co(2-(3',4'-bis(2-methylpropyloxy)phenyl)-p-phenylenevinylene),
poly-2-(3'-(3,7-dimethyloctyloxy)phenyl)-p-phenylenevinylene,
2-(2',5'-dimethylphenyl)poly-p-phenylenevinylene, and
poly-(4'-hexyloxyphenyl)phenylenevinylene-co-alt-(2-(3',7'-dimethyloctyloxy)-5-methoxy)phenylenevinylene.

2. An electroluminescent device as claimed in Claim 1, **characterized in that** the aryl-1,4-phenylene unit is a unit of the formula (C1) or (C2) 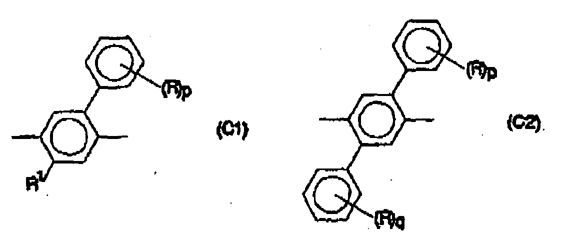 wherein
R is independently selected each time it occurs and represents C₁-C₂₀ alkyl or C₁-C₂₀ alkoxy, R¹ represents hydrogen or C₁-C₂₀ alkoxy,
p and q are 0, 1, 2, 3, 4 or 5, and
one or more non-neighboring -CH₂- units of a C₁-C₂₀ alkyl or C₁-C₂₀ alkoxy may be replaced by -O-, -S-, -N(R²)-, wherein R² equals phenyl or C₁-C₂₀ alkyl, phenylene, and/or-COO.

3. An electroluminescent device as claimed in Claim 2, **characterized in that** the poly(1,4-phenylenevinylene) substantially consists of an aryl-1,4-phenylenevinylene and a 2,5-dialkoxy-1,4-phenylenevinylene repeating unit.

4. An electroluminescent device as claimed in any one of the Claims 1, 2 or 3, **characterized in that** the aryl-1,4-phenylene units are present in a proportion of 0.95 to 1.0 inclusive.

5. A soluble poly(1,4-phenylenevinylene) wherein at least one 1,4-phenylene unit is an aryl-1,4-phenylene unit, where the aryl group represents a phenyl, naphthyl, or biphenylyl group which may or may not be substituted, with the exception of the copolymer poly((3-methoxyphenyl)-1,4-phenylenevinylene-co-2-methoxy-5-(2-(trimethylammonium iodide)-ethoxy)-1,4-phenylenevinylene) and poly(p-phenylphenylene vinylene),
and with the exclusion of
poly(2-methoxy-5-(3,7-dimethyloetyloxy)-p-phenylenevinylene)co-(2-(4'-(3,7-dimethyloctyloxy)phenyl)-p-phenylenevinylene),
poly(2-methoxy-5-(3,7-dimethyloctyloxy)-p-phenylenevinylene)co-(2-(3'-(3,7-dimethyloctyloxy)phenyl)-p-phenylenevinylene),
poly(2-methoxy-5-(3,7-dimethyloctyloxy)-p-phenylenevinylene)co-(2-(2',5'-dimethyl)phenyl)-p-phenylenevinylene),
poly(2-methoxy-5-(3,7-dimethyloctyloxy)-p-phenylenevinylene)co-(2-(3'-(3,7-dimethyloctyloxy)phenyl)-p-phenylenevinylene)co-(2-(2',5'-dimethyl)phenyl)-p-phenylenevinylene),
paly(2,5-bis(3,7-dimethyloctyloxy)-p-phenylenevinylene)co(2-(3'-(3,7-dimethyloctyloxy)phenyl)-p-phenylenevinylene)phenyleneylene)co(2-(4'-(3,7-dimethyloctyloxy)phenyl)-p-phenylenevinylene)phenylenevinylene),
poly(2-(3,7-dimethyloctyloxy)-5-methoxy-p-phenylenevinylene)co(2-(3'-(3,7-dimethyloctyloxy)phenyl)-5-methoxy-p-phenylenevinylene),
poly(2-(3'-(3,7-dimethyloetyloxy)phenyl)-p-phenylenevinylene)co(2-(4'-(3,7-dimethyloctyloxy)phenyl)-p-phenylenevinylene),
poly(2-(4'-(3,7-dimethyloctyloxy)phenyl)-p-phenylenevinylene)co(2-(2',5-dimethyl)phenyl)-p-phenylenevinylene),
poly(2-methoxy-5-(3,7-dimethyloctyloxy)-p-phenylenevinylene)co(2-(3'-(3,7-dimethyloctyloxy)phenyl)-p-phenylenevinylene)phenylenevinylene)co(2-(4'-(3,7-dimethyloctyloxy)phenyl)-p-phenylenevinylene)co(2-(2',5'-dimethyl)phenyl)-p-phenylene,
poly(2-(3'-(3,7-dimethyloctyloxy)phenyl)-p-phenylenevinylene)co(2-(3',4'-bis(2-methylpropyloxy)phenyl)-p-phenylenevinylene),
poly-2-(3'-(3,7-dimethyloctyloxy)phenyl)-p-phenylenevinylene,
2-(2',5'-dimethylphenyl)poly-p-phenylenevinylene, and
poly-(4'-hexyloxyphenyl)phenylenevinylene-co-alt-(2-(3',7'-dimethyloctyloxy)-5-methoxy)phenylenevinylene.

6. Ain intermediate compound suitable for the preparation of a poly(1,4-phenylenevinylene) as claimed in Claim 5, said intermediate compound being an aryl-1,4-bis(halomethyl)benzene, wherein the aryl group represents a phenyl, naphthyl, or biphenylyl group which may or may not be substituted, with the exception of 2-phenyl-1,4-bis(bromomethyl)benzene and 2-(3-methoxyphenyl)-1,4-bis(chloromethyl)benzene,
and with the exclusion of
2,5-bisbrommethyl-4'-hexyloxybiphenyl,
2,5-bischlormethyl-4'-hexyloxybiphenyl,
2,5-bisbrommethyl-2',5'-dimethylbiphenyl,
2,5-bischlormethyl-7',5'-dimethylbiphenyl,
2,5-bischlormethyl-3'-(3,7-dimethyloctyloxy)biphenyl
2,5-bischlormethyl-4'-(3,7-dimethyloctyloxy)biphenyl,
2,5-bischlormethyl-3',4'-bis(2-methylpropyloxy)biphenyl,
2,5-bischlormethyl-4"-(3,7-dimethyloctyloxy)terphenyl,
2,5-bischlormethyl-4-methoxy-4'-(3,7-dimethyloctyloxy)-biphenyl, and
2,5-bischlormethyl-4-methoxy-3'-(3,7-dimethyloctyloxy)-bipbenyl.

## Patentansprüche

1. Elektrolumineszenzvorrichtung enthaltend eine zwischen einer loch injizierenden und einer elektroneninjizierenden Elektrode befindliche Elektrolumineszenzschicht, wobei die Elektrolumineszenzschicht ein lösliches Poly(1,4-phenylenvinylen) enthält, **dadurch gekennzeichnet, dass** es sich bei mindestens einer 1,4-Phenylen-Einheit des Poly(1,4-phenylenvinylen)s um eine Aryl-1,4-phenylen-Einheit handelt, worin die Arylgruppe eine Phenyl-, Naphthyl- oder Biphenylylgruppe bedeutet, die gegebenenfalls substituiert sein kann, mit Ausnahme des Copolymers Poly(2-(3-methoxyphenyl)-1,4-phenylenvinylen-co-2-methoxy-5-(2-(trimethylammoniumiodid)-ethoxy)-1,4-phenylenvinylen) und Poly(p-phenylphenylenvinylen),
und unter Ausschluss von
Poly(2-methoxy-5-(3,7-dimethyloctyloxy)-p-phenylenvinylen)co-(2-(4'-(3,7-dimethyloctyloxy)phenyl)-p-phenylenvinylen),
Poly(2-methoxy-5-(3,7-dimethyloctyloxy)-p-phenylenvinylen)co-(2-(3'-(3,7-dimethyloctyloxy)phenyl)-p-phenylenvinylen),
Poly(2-methoxy-5-(3,7-dimethyloctyloxy)-p-phenylenvinylen)co-(2-(2',5'-dimethyl)phenyl)-p-phenylenvinylen),
Poly(2-methoxy-5-(3,7-dimethyloctyloxy)-p-phenylenvinylen)co-(2-(3'-(3,7-dimethyloctyloxy)phenyl)-p-phenylenvinylen)co-(2-(2',5'-dimethyl)phenyl)-p-phenylenvinylen),
Poly(2,5-bis(3,7-dimethyloctyloxy)-p-phenylenvinylen)co(2-(3'-(3,7-dimethyloctyloxy)phenyl)-p-phenylenvinylen)phenylenvinylen)co(2-(4'-(3,7-dimethyloctyloxy)phenyl)-p-phenylenvinylen)phenylenvinylen),
Poly(2-(3,7-dimethyloctyloxy)-5-methoxy-p-phenylenvinylen)co(2-(3'-(3,7-dimethyloctyloxy)phenyl)-5-methoxy-p-phenylenvinylen),
Poly(2-(3'-(3,7-dimethyloctyloxy)phenyl)-p-phenylenvinylen)co(2-(4'-(3,7-dimethyloctyloxy)phenyl)-p-phenylenvinylen),
Poly(2-(4'-(3,7-dimethyloctyloxy)phenyl)-p-phenylenvinylen)co(2-(2',5'-dimethyl)phenyl)-p-phenylenvinylen),
Poly(2-methoxy-5-(3,7-dimethyloctyloxy)-p-phenylenvinylen)co(2-(3'-(3,7-dimethyloctyloxy)phenyl)-p-phenylenvinylen)phenylenvinylen)co(2-(4'-(3,7-dimethyloctyloxy)phenyl)-p-phenylenvinylen)co(2-(2',5'-dimethyl)phenyl)-p-phenylen,
Poly(2-(3'-(3,7-dimethyloctyloxy)phenyl)-p-phenylenvinylen)co(2-(3',4'-bis(2-methylpropyloxy)phenyl)-p-phenylenvinylen),
Poly-2-(3'-(3,7-dimethyloctyloxy)phenyl)-p-phenylenvinylen,
2-(2',5'-Dimethylphenyl)poly-p-phenylenvinylen und
Poly-(4'-hexyloxyphenyl)phenylenvinylen-co-alt-(2-(3',7'-dimethyloctyloxy)-5-methoxy)phenylenvinylen.

2. Elektrolumineszenzvorrichtung nach Anspruch 1, **dadurch gekennzeichnet, dass** es sich bei der Aryl-1,4-phenylen-Einheit um eine Einheit der Formel (C1) oder (C2) 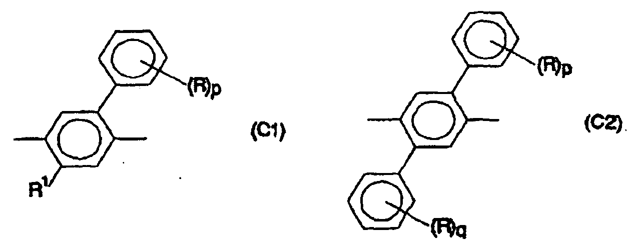 handelt, worin
R bei jedem Auftreten unabhängig ausgewählt ist und C₁C₂₀-Alkyl oder C₁C₂₀-Alkoxy bedeutet, R¹ Wasserstoff oder C₁-C₂₀-Alkoxy bedeutet,
p und q 0, 1, 2, 3, 4 oder 5 bedeuten, und
eine oder mehrere nicht benachbarte -CH₂-Einheiten eines C₁-C₂₀-Alkyl oder C₁-C₂₀-Alkoxy durch -O-, -S-, -N(R²)-, worin R² gleich Phenyl oder C₁-C₂₀-Alkyl ist, Phenylen und/oder -COO- ersetzt sein können.

3. Elektrolumineszenzvorrichtung nach Anspruch 2, **dadurch gekennzeichnet, dass** das Poly(1,4-phenylenvinylen) im Wesentlichen aus einer Aryl-1,4-phenylenvinylen- und einer 2,5-Dialkoxy-1,4-phenylenvinylen-Wiederholungseinheit besteht.

4. Elektrolumineszenzvorrichtung nach einem beliebigen der Ansprüche 1, 2 oder 3, **dadurch gekennzeichnet, dass** die Aryl-1,4-phenylen-Einheiten in einem Anteil von 0,95 bis einschließlich 1,0 vorliegen.

5. Lösliches Poly(1,4-phenylenvinylen), bei dem es sich bei mindestens einer 1,4-Phenylen-Einheit um eine Aryl-1,4-phenylen-Einheit handelt, worin die Arylgruppe eine Phenyl-, Naphthyl- oder Biphenylylgruppe bedeutet, die gegebenenfalls substituiert sein kann, mit Ausnahme des Copolymers Poly((3-methoxyphenyl)-1,4-phenylenvinylen-co-2-methoxy-5-(2-(trimethylammoniumiodid)-ethoxy)-1,4-phenylenvinylen) und Poly(p-phenylphenylenvinylen),
und unter Ausschluss von
Poly(2-methoxy-5-(3,7-dimethyloctyloxy)-p-phenylenvinylen)co-(2-(4'-(3,7-dimethyloctyloxy)phenyl)-p-phenylenvinylen),
Poly(2-methoxy-5-(3,7-dimethyloctyloxy)-p-phenylenvinylen)co-(2-(3'-(3,7-dimethyloctyloxy)phenyl)-p-phenylenvinylen),
Poly(2-methoxy-5-(3,7-dimethyloctyloxy)-p-phenylenvinylen)co-(2-(2',5'-dimethyl)phenyl)-p-phenylenvinylen),
Poly(2-methoxy-5-(3,7-dimethyloctyloxy)-p-phenylenvinylen)co-(2-(3'-(3,7-dimethyloctyloxy)phenyl)-p-phenylenvinylen)co-(2-(2',5'-dimethyl)phenyl)-p-phenylenvinylen),
Poly(2,5-bis(3,7-dimethyloctyloxy)-p-phenylenvinylen)co(2-(3'-(3,7-dimethyloctyloxy)phenyl)-p-phenylenvinylen)phenylenvinylen)co(2-(4'-(3,7-dimethyloctyloxy)phenyl)-p-phenylenvinylen)phenylenvinylen),
Poly(2-(3,7-dimethyloctyloxy)-5-methoxy-p-phenylenvinylen)co(2-(3'-(3,7-dimethyloctyloxy)phenyl)-5-methoxy-p-phenylenvinylen),
Poly(2-(3'-(3,7-dimethyloctyloxy)phenyl)-p-phenylenvinylen)co(2-(4'-(3,7-dimethyloctyloxy)phenyl)-p-phenylenvinylen),
Poly(2-(4'-(3,7-dimethyloctyloxy)phenyl)-p-phenylenvinylen)co(2-(2',5'-dimethyl)phenyl)-p-phenylenvinylen),
Poly(2-methoxy-5-(3,7-dimethyloctyloxy)-p-phenylenvinylen)co(2-(3'-(3,7-dimethyloctyloxy)phenyl)-p-phenylenvinylen)phenylenvinylen)co(2-(4'-(3,7-dimethyloctyloxy)phenyl)-p-phenylenvinylen)co(2-(2',5'-dimethyl)phenyl)-p-phenylen,
Poly(2-(3'-(3,7-dimethyloctyloxy)phenyl)-p-phenylenvinylen)co(2-(3',4'-bis(2-methylpropyloxy)phenyl)-p-phenylenvinylen),
Poly-2-(3'-(3,7-dimethyloctyloxy)phenyl)-p-phenylenvinylen,
2-(2',5'-Dimethylphenyl)poly-p-phenylenvinylen und
Poly-(4'-hexyloxyphenyl)phenylenvinylen-co-alt-(2-(3',7'-dimethyloctyloxy)-5-methoxy)phenylenvinylen.

6. Zwischenverbindung geeignet für die Herstellung eines Poly(1,4-phenylenvinylen) nach Anspruch 5, wobei es sich bei der Zwischenverbindung um ein Aryl-1,4-bis(halogenmethyl)benzol handelt, worin die Arylgruppe eine Phenyl-, Naphthyl- oder Biphenylylgruppe bedeutet, die gegebenenfalls substituiert sein kann, mit Ausnahme von 2-Phenyl-1,4-bis(brommethyl)benzol und 2-(3-Methoxyphenyl)-1,4-bis(chlormethyl)benzol,
und unter Ausschluss von
2,5-Bisbrommethyl-4'-hexyloxybiphenyl,
2,5-Bischlormethyl-4'-hexyloxybiphenyl,
2,5-Bisbrommethyl-2',5'-dimethylbiphenyl,
2,5-Bischlormethyl-2',5'-dimethylbiphenyl,
2,5-Bischlormethyl-3'-(3,7-dimethyloctyloxy)biphenyl,
2,5-Bischlormethyl-4'-(3,7-dimethyloctyloxy)biphenyl,
2,5-Bischlormethyl-3',4'-bis(2-methylpropyloxy)biphenyl,
2,5-Bischlormethyl-4"-(3,7-dimethyloctyloxy)terphenyl,
2,5-Bischlormethyl-4-methoxy-4'-(3,7-dimethyloctyloxy)-biphenyl und
2,5-Bischlormethyl-4-methoxy-3'-(3,7-dimethyloctyloxy)-biphenyl.

## Revendications

1. Dispositif électroluminescent comprenant une couche électroluminescente disposée entre une électrode d'injection de trous et une électrode d'injection d'électrons, ladite couche électroluminescente comprenant un poly(1,4-phénylènevinylène) soluble, **caractérisé en ce qu'**au moins une unité 1,4-phénylène dudit poly(1,4-phénylènevinylène) est une unité aryle-1,4-phénylène, dans laquelle le groupe aryle représente un groupe phényle, naphtyle, ou biphénylyle qui peut être ou ne pas être substitué, à l'exception du copolymère poly(2-(3-méthoxyphényle)-1,4-phénylènevinylène-co-2-méthoxy-5-(2-(iodure de triméthylammonium)-éthoxy)-1,4-phénylènevinylène) et poly(p-phénylphénylène vinylène),
et à l'exclusion de
poly(2-méthoxy-5-(3,7-diméthyloctyloxy)-p-phénylènevinylène)co-(2-(4'-(3,7-diméthyloctyloxy)phényl)-p-phénylènevinylène),
poly(2-méthoxy-5-(3,7-diméthyloctyloxy)-p-phénylènevinylène)co-(2-(3'-(3,7-diméthyloctyloxy)phényl)-p-phénylènevinylène),
poly(2-méthoxy-5-(3,7-diméthyloctyloxy)-p-phénylènevinylène)co-(2-(2',5'-diméthyl)phényl)-p-phénylènevinylène),
poly(2-méthoxy-5-(3,7-diméthyloctyloxy)-p-phénylènevinylène)co-(2-(3'-(3,7-diméthyloctyloxy)phényl)-p-phénylènevinylène)co-(2-(2',5'-diméthyl)phényl)-p-phénylènevinylène),
poly(2,5-bis(3,7-diméthyloctyloxy)-p-phénylènevinylène)co(2-(3'-(3,7-diméthyloctyloxy)phényl)-p-phénylènevinylène)phénylènevinylène)co(2-(4'-(3, 7-diméthyloctyloxy)phényl)-p-phénylènevinylène)phénylènevinylène),
poly(2-(3,7-diméthyloctyloxy)-5-méthoxy-p-phénylènevinylène)co(2-(3'-(3,7-diméthyloctyloxy)phényl)-5-méthoxy-p-phénylènevinylène),
poly(2-(3'-(3,7-diméthyloctyloxy)phényl)-p-phénylènevinylène)co(2-(4'-(3,7-diméthyloctyloxy)phényl)-p-phénylènevinylène),
poly(2-(4'-(3,7-diméthyloctyloxy)phényl)-p-phénylènevinylène)co(2-(2',5'-diméthyl)phényl)-p-phénylènevinylène),
poly(2-méthoxy-5-(3,7-diméthyloctyloxy)-p-phénylènevinylène)co(2-(3'-(3,7-diméthyloctyloxy)phényl)-p-phénylènevinylèné)phénylènevinylène)co(2-(4'-(3,7-diméthyloctyloxy)phényl)-p-phénylènevinylène)co(2-(2',5'-diméthyl)phényl)-p-phénylène,
poly(2-(3'-(3,7-diméthyloctyloxy)phényl)-p-phénylènevinylène)co(2-(3',4'-bis(2-méthylpropyloxy)phényl)-p-phénylènevinylène),
poly-2-(3'-(3,7-diméthyloctyloxy)phényl)-p-phénylènevinylène,
2-(2',5'-diméthylphényl)poly-p-phénylènevinylène, et
poly-(4'-hexyloxyphényl)phénylènevinylène-co-alt-(2-(3',7'-diméthyloctyloxy)-5-méthoxy)phénylènevinylène.

2. Dispositif électroluminescent selon la revendication 1, **caractérisé en ce que** l'unité aryle-1,4-phénylène est une unité de la formule (C1) ou (C2) 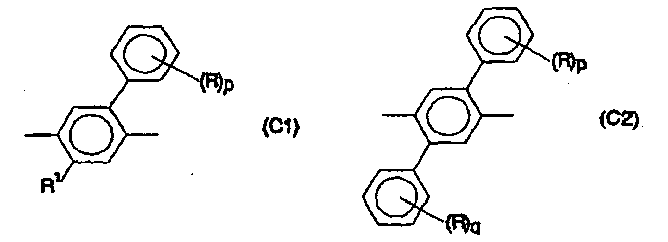 dans laquelle
R est choisi indépendamment chaque fois qu'il se trouve et représente C₁-C₂₀ alkyle ou C₁-C₂₀ alcoxy, R¹ représente hydrogène ou C₁-C₂₀ alcoxy,
p et q sont 0, 1, 2, 3, 4 ou 5, et
une ou plusieurs unités -CH₂- non voisines d'un C₁-C₂₀ alkyle ou C₁-C₂₀ alcoxy peuvent être remplacées par -O-, -S-, -N(R²)-, dans lequel R² est égal à phényle ou C₁-C₂₀ alkyle, phénylène, et/ou -COO-.

3. Dispositif électroluminescent selon la revendication 2, **caractérisé en ce que** le poly(1,4-phénylènevinylène) est constitué essentiellement d'un aryle-1,4-phénylènevinylène et d'une unité se répétant 2,5-dialcoxy-1,4-phénylènevinylène.

4. Dispositif électroluminescent selon l'une quelconque des revendications 1, 2 ou 3, **caractérisé en ce que** les unités aryle-1,4-phénylène sont présentes dans une proportion de 0,95 à 1,0, bornes incluses.

5. Poly(1,4-phénylènevinylène) soluble dans lequel au moins une unité 1,4-phénylène est une unité aryle-1,4-phénylène, dans laquelle le groupe aryle représente un groupe phényle, naphtyle, ou biphénylyle qui peut être ou ne pas être substitué, à l'exception du copolymère poly((3-méthoxyphényl)-1,4-phénylènevinylène-co-2-méthoxy-5-(2-(iodure de triméthylammonium)-éthoxy)-1,4-phénylènevinylène) et poly(p-phénylphénylène vinylène),
et à l'exclusion de
poly(2-méthoxy-5-(3,7-diméthyloctyloxy)-p-phénylènevinylène)co-(2-(4'-(3,7-diméthyloctyloxy)phényl)-p-phénylènevinylène),
poly(2-méthoxy-5-(3,7-diméthyloctyloxy)-p-phénylènevinylène)co-(2-(3'-(3,7-diméthyloctyloxy)phényl)-p-phénylènevinylène),
poly(2-méthoxy-5-(3,7-diméthyloctyloxy)-p-phénylènevinylène)co-(2-(2',5'-diméthyl)phényl)-p-phénylènevinylène),
poly(2-méthoxy-5-(3,7-d iméthyloctyloxy)-p-phénylènevinylène)co-(2-(3'-(3,7-diméthyloctyloxy)phényl)-p-phénylènevinylène)co-(2-(2',5'-diméthyl)phényl)-p-phénylènevinylène),
poly(2,5-bis(3,7-diméthyloctyloxy)-p-phénylènevinylène)co(2-(3'-(3,7-diméthyloctyloxy)phényl)-p-phénylènevinylène)phénylènevinylène)co(2-(4'-(3,7-diméthyloctyloxy)phényl)-p-phénylènevinylène)phénylènevinylène),
poly(2-(3,7-diméthyloctyloxy)-5-méthoxy-p-phénylènevinylène)co(2-(3'-(3,7-diméthyloctyloxy)phényl)-5-méthoxy-p-phénylènevinylène),
poly(2-(3'-(3,7-diméthyloctyloxy)phényl)-p-phénylènevinylène)co(2-(4'-(3,7-diméthyloctyloxy)phényl)-p-phénylènevinylène),
poly(2-(4'-(3,7-diméthyloctyloxy)phényl)-p-phénylènevinylène)co(2-(2',5'-diméthyl)phényl)-p-phénylènevinylène),
poly(2-méthoxy-5-(3,7-diméthyloctyloxy)-p-phénylènevinylène)co(2-(3'-(3,7-diméthyloctyloxy)phényl)-p-phénylènevinylène)phénylènevinylène)co(2-(4'-(3,7-diméthyloctyloxy)phényl)-p-phénylènevinylène)co(2-(2',5'-diméthyl)phényl)-p-phénylène,
poly(2-(3'-(3,7-diméthyloctyloxy)phényl)-p-phénylènevinylène)co(2-(3',4'-bis(2-méthylpropyloxy)phényl)-p-phénylènevinylène),
poly-2-(3'-(3,7-diméthyloctyloxy)phényl)-p-phénylènevinylène,
2-(2',5'-diméthylphényl)poly-p-phénylènevinylène, et
poly-(4'-hexyloxyphényl)phénylènevinylène-co-alt-(2-(3',7'-diméthyloctyloxy)-5-méthoxy)phénylènevinylène.

6. Composé intermédiaire convenant pour la préparation d'un poly(1,4-phénylènevinylène) selon la revendication 5, ledit composé intermédiaire étant un aryle-1,4-bis(halométhyl)benzène, dans lequel le groupe aryle représente un groupe phényle, naphtyle, ou biphénylyle qui peut être ou ne pas être substitué, à l'exception de 2-phényle-1,4-bis(bromométhyl)benzène et 2-(3-méthoxyphényl)-1,4-bis(chlorométhyl)benzène,
et à l'exclusion de
2,5-bisbromméthyle-4'-hexyloxybiphényle,
2,5-bischlorméthyle-4'-hexyloxybiphényle,
2,5-bisbromméthyle-2',5'-diméthylbiphényle,
2,5-bischlorméthyle-2',5'-diméthylbiphényle,
2,5-bischlorméthyle-3'-(3,7-diméthyloctyloxy)biphényle,
2,5-bischlorméthyle-4'-(3,7-diméthyloctyloxy)biphényle,
2,5-bischlorméthyle-3',4'-bis(2-méthylpropyloxy)biphényle,
2,5-bischlorméthyle-4"-(3,7-diméthyloctyloxy)terphényle,
2,5-bischlorméthyle-4-méthoxy-4'-(3,7-diméthyloctyloxy)-biphényle, et
2,5-bischlorméthyle-4-méthoxy-3'-(3,7-diméthyloctyloxy)-biphényle.
